# DEMANDE DE BREVET EUROPEEN

(11) **EP 1 036 560 A1**
(43) Date de publication de la demande: **20.09.2000**
(21) Numéro de dépôt: 00400098.0
(22) Date de dépôt: 14.01.2000
(51) Int. Cl.: A61K 7/48, A61K 7/06, A61K 7/42

(54) **Composition fluide sous forme d'émulsion huile-dans-eau contenant un terpolymère acrylique et ses utilisations notamment cosmétiques**

(30) Priorité: 17.03.1999 FR 9903319
(71) Demandeur: L'OREAL, 75008 Paris (FR)
(72) Inventeur: Simon, Pascal, 94400 Vitry sur Seine (FR)
(74) Mandataire: Rasson, Catherine

(57) **Abrégé**

La présente demande se rapporte à une composition fluide sous forme d'émulsion huile-dans-eau, comprenant dans un milieu physiologiquement acceptable, une phase huileuse dispersée dans une phase aqueuse et contenant jusqu'à 0,5 % en poids de matière active par rapport au poids total de la composition, d'au moins un terpolymère acrylique obtenu à partir d'un acide carboxylique à insaturation α,β-éthylénique, d'un monomère à insaturation éthylénique non tensioactif différent de (a), et d'un monomère uréthanne non-ionique qui est le produit de réaction d'un composé amphiphile non ionique monohydrique avec un isocyanate à insaturation monoéthylénique.

La composition obtenue est avantageusement exempte de tout tensioactif classiquement utilisé dans les émulsions H/E. Elle a une très bonne stabilité et est assez fluide pour être appliquée au moyen d'un vaporisateur, lequel est exempt de gaz propulseur, est muni d'une pompe type pompe spray, fonctionne à la pression atmosphérique et délivre la composition sous forme de fines gouttelettes.

L'invention se rapporte aussi à l'utilisation de la dite composition, notamment pour le soin, le démaquillage, le nettoyage et/ou le parfumage de la peau, des cheveux et/ou des lèvres. Elle peut servir aussi pour l'imprégnation de tissus destinés au nettoyage et/ou au démaquillage de la peau, des lèvres et/ou des yeux.

## Description

La présente demande se rapporte à une composition fluide sous forme d'émulsion huile-dans-eau contenant au moins un terpolymère acrylique, et à l'utilisation de la dite composition, notamment pour le soin, le démaquillage, le nettoyage et/ou le parfumage de la peau du corps ou du visage, des cheveux et/ou des lèvres et pour l'imprégnation de tissus destinés au nettoyage et/ou au démaquillage de la peau, des lèvres et/ou des yeux.

Pour diverses raisons liées en particulier à un meilleur confort d'utilisation (douceur, émollience et autres), les compositions cosmétiques actuelles se présentent le plus souvent sous la forme d'une émulsion du type huile-dans-eau (H/E) constituée d'une phase continue dispersante aqueuse et d'une phase discontinue dispersée huileuse ou d'une émulsion du type eau-dans-huile (E/H) constituée d'une phase continue dispersante huileuse et d'une phase discontinue dispersée aqueuse. Les émulsions H/E sont les plus demandées dans le domaine cosmétique du fait qu'elles comportent comme une phase externe, une phase aqueuse, ce qui leur confère, lors de l'application sur la peau, un toucher plus frais, moins gras et plus léger que les émulsions E/H.

Les émulsions sont généralement stabilisées par des tensioactifs émulsionnants appropriés qui, grâce à leur structure amphiphile, se placent à l'interface huile/eau et stabilisent ainsi les gouttelettes dispersées. Ces émulsionnants présentent cependant l'inconvénient d'être pénétrants et potentiellement irritants pour la peau, les yeux et le cuir chevelu, notamment pour les sujets à peau sensible.

En outre, dans ces émulsions classiques qui contiennent des tensioactifs émulsionnants, la taille des globules huileux est généralement supérieure à plusieurs microns. De telles émulsions peuvent avoir des propriétés cosmétiques et physico-chimiques insuffisantes (toucher huileux, instabilité dans le temps). Le fait d'augmenter le taux des tensioactifs ne résout pas généralement les problèmes mentionnés. La stabilité requise n'est pas toujours atteinte et les propriétés cosmétiques ne sont pas améliorées (toucher cireux, lourd, manque de fraîcheur à l'application). Par ailleurs, comme indiqué ci-dessus, il est aussi déconseillé d'utiliser de trop fort taux de tensioactif pour des raisons d'innocuité.

Une solution pour s'affranchir des phénomènes d'instabilité des émulsions H/E (crémage et déphasage) consiste à ajouter dans l'émulsion des agents épaississants dont la fonction est de créer, au sein de la phase aqueuse, une matrice gélifiée servant à figer les gouttelettes huileuses et assurant un maintien mécanique de l'ensemble de l'émulsion. Toutefois, cette solution présente l'inconvénient de ne pas permettre d'obtenir toutes les textures désirées et en particulier les textures fluides, légères, s'appliquant facilement et rapidement sur la peau sans laisser de film résiduel.

Par ailleurs, il a été envisagé de remplacer les tensioactifs par des polymères comportant dans leur chaîne une partie hydrophile et une partie hydrophobe constituée d'une chaîne grasse, tels que les copolymères d'alkyl-C₁₀-C₃₀-acrylate et d'acide acrylique ou méthacrylique, comme le produit "PEMULEN TR2" commercialisé par la société Goodrich. Cependant, ces polymères présentent l'inconvénient de ne pas permettre l'obtention de compositions suffisamment fluides pour être vaporisables.

L'objectif de l'invention est de pouvoir réaliser des émulsions huile-dans-eau très fluides et stables, ne contenant éventuellement pas de tensioactif émulsionnant classiquement utilisé dans les émulsions H/E et présentant de bonnes propriétés cosmétiques sans avoir les inconvénients de l'art antérieur.

La demanderesse a découvert de façon inattendue une nouvelle famille de polymères permettant de réaliser de telles émulsions.

Ces polymères permettent de préparer des émulsions huile-dans-eau, dans une large gamme de viscosité et de teneur en phase huileuse, qui restent stables dans le temps à température ambiante ou à des températures plus élevées.

Ainsi, la présente invention concerne une composition fluide sous forme d'émulsion huile-dans-eau comprenant dans un milieu physiologiquement acceptable, une phase huileuse dispersée dans une phase aqueuse, caractérisée par le fait qu'elle contient jusqu'à 0,5 % en poids de matière active par rapport au poids total de la composition, d'au moins un terpolymère acrylique obtenu à partir (a) d'un acide carboxylique à insaturation α,β-éthylénique, (b) d'un monomère à insaturation éthylénique non tensioactif différent de (a), et (c) d'un monomère uréthanne non-ionique qui est le produit de réaction d'un composé amphiphile non-ionique monohydrique avec un isocyanate à insaturation monoéthylénique.

La composition obtenue a une texture homogène et agréable à l'application tout en étant stable à la conservation. Par ailleurs, elle est très fluide. La viscosité des émulsions va généralement de 0,02 à 3 Pa.s, cette viscosité étant mesurée à environ 25°C à l'aide du viscosimètre "Rhéomat 180" équipé d'un mobile 2.

Avantageusement, l'émulsion de l'invention est exempte de tensioactif classiquement utilisé dans les H/E et elle présente de ce fait l'avantage de ne pas être irritante pour les peaux particulièrement sensibles. En outre, cette émulsion présente l'avantage de permettre l'incorporation d'actifs thermosensibles car elle peut être fabriquée à température ambiante, à la fois dans l'étape de neutralisation du polymère et dans l'étape de dispersion de la phase huileuse.

Le terpolymère acrylique utilisé conformément à l'invention est soluble ou gonflable dans les alcalins. Il est de préférence caractérisé par le fait qu'il comprend, par rapport au poids total du terpolymère, :
(a) environ 20 à 70 % en poids, de préférence 25 à 55 % en poids, d'un acide carboxylique à insaturation α,β-monoéthylénique ;
(b) environ 20 à 80 % en poids, de préférence 30 à 65 % en poids, d'un monomère à insaturation monoéthylénique non tensioactif différent de (a) ; et
(c) environ 0,5 à 60 % en poids, de préférence 10 à 50 % en poids, d'un monomère uréthane non-ionique qui est le produit de réaction d'un composé amphiphile non-ionique monohydrique avec un isocyanate à insaturation monoéthylénique.

L'acide carboxylique à insaturation α,β-monoéthylénique (a) peut être choisi parmi de nombreux acides et en particulier l'acide acrylique, l'acide méthacrylique, l'acide crotonique, l'acide itaconique et l'acide maléique. Il s'agit de préférence de l'acide méthacrylique. Une large proportion d'acide est préférable pour donner une structure polymère qui se solubilise et donne un agent épaississant par réaction avec un composé alcalin comme l'hydroxyde de sodium, les alcanolamines, l'aminométhyl-propanol, ou l'aminométhyl-propanediol.

Le terpolymère contient un monomère (b) à insaturation monoéthylénique qui n'a pas de propriété tensioactive et qui est présent aussi de préférence en une proportion importante telle qu'indiquée ci-dessus. Les monomères préférés sont ceux qui donnent des polymères insolubles dans l'eau lorsqu'ils sont homopolymérisés et ils sont illustrés par les acrylates et méthacrylates d'alkyle en C₁-C₄ comme l'acrylate de méthyle, l'acrylate d'éthyle, l'acrylate de butyle ou les méthacrylates correspondants. Les monomères plus particulièrement préférés sont l'acrylate de méthyle et l'acrylate d'éthyle. D'autres monomères pouvant être utilisés sont le styrène, le vinyltoluène, l'acétate de vinyle, l'acrylonitrile et le chlorure de vinylidène. Les monomères non réactifs sont préférés, ces monomères étant ceux dans lesquels le groupe éthylénique unique est le seul groupe réactif dans les conditions de la polymérisation. Toutefois, des monomères qui contiennent des groupes réactifs sous l'action de la chaleur comme l'acrylate d'hydroxyéthyle peuvent éventuellement être utilisés.

Les composés amphiphiles non-ioniques monohydriques utilisés pour obtenir le monomère uréthane non-ionique (c) sont bien connus et sont généralement des composés hydrophobes alcoxylés contenant un oxyde d'alkylène formant la partie hydrophile de la molécule. Les composés hydrophobes sont généralement constitués par un alcool aliphatique ou un alkylphénol dans lesquels une chaîne carbonée contenant au moins six atomes de carbone constitue la partie hydrophobe du composé amphiphile.

Les composés amphiphiles non-ioniques monohydriques préférés sont des composés ayant la formule (I) suivante :

R-(OCH₂CHR')ₘ-(OCH₂CH₂)ₙ-OH (I)

dans laquelle R est choisi parmi les groupes alkyle comportant de 6 à 30 atomes de carbone et les groupes aralkyle ayant des radicaux alkyle comportant de 8 à 30 atomes de carbone, R' est choisi parmi les groupes alkyle comportant de 1 à 4 atomes de carbone, n est un nombre moyen allant d'environ 6 à 150 et m est un nombre moyen allant d'environ 0 à 50, à la condition que n soit au moins aussi grand que m et que n + m = 6 à 150.

De préférence, dans les composés de formule (I), le groupe R est choisi parmi les groupes alkyle comportant de 18 à 26 atomes de carbone et les groupes alkyl-(C₈-C₁₃)-phényle ; le groupe R' est le groupe méthyle ; m = 0 et n = 6 à 150. Le composé de formule (I) peut en particulier être un dérivé oxyalkyléné, en particulier oxyéthyléné, d'un alcool aliphatique d'origine végétale et notamment de l'alcool béhénylique, le radical R dans la formule (I) étant alors le radical béhényle.

L'isocyanate à insaturation monoéthylénique utilisé pour former le monomère uréthane non-ionique (c) peut être choisi parmi des composés très variés. On peut utiliser un composé contenant toute insaturation copolymérisable telle qu'une insaturation acrylique, méthacrylique ou allylique. L'isocyanate à insaturation monoéthylénique préféré est l'α,α-diméthyl-m-isopropényl-benzyl-isocyanate.

Le terpolymère acrylique défini ci-dessus est obtenu par copolymérisation en dispersion aqueuse des composants (a), (b) et (c), copolymérisation qui est tout à fait usuelle et décrite notamment dans le document EP-A-0 173 109.

A titre de terpolymères pouvant être utilisés selon l'invention, on peut citer le produit de réaction de l'acide méthacrylique comme composant (a), de l'acrylate d'éthyle comme composant (b) et d'un macromonomère uréthane non-ionique comme composant (c), ayant la structure (II) suivante : 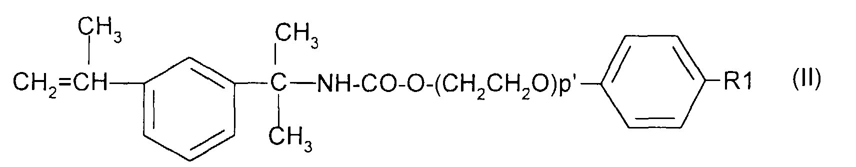 dans laquelle p' va de 6 à 150 et est égal de préférence à 30 et R1 est choisi parmi les radicaux alkyle comportant de 8 à 13 atomes de carbone, tel que décrit dans l'exemple 3 du document EP-A-0 173 109.

Le terpolymère acrylique préféré utilisé selon l'invention est obtenu à partir d'acide méthacrylique comme composant (a), d'acrylate de méthyle comme composant (b) et d'un macromonomère uréthane non-ionique comme composant (c), ayant la structure (III) suivante : 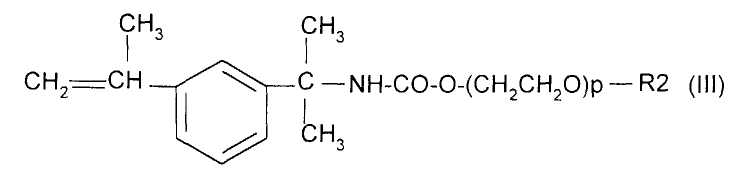 dans laquelle p va de 6 à 150 et R2 est choisi parmi les radicaux alkyle linéaires comportant de 18 à 26 et de préférence de 20 à 24 atomes de carbone. De préférence, le radical R2 dans la composé de formule (III) est un radical d'origine végétale, tel que le radical béhényle.

Les terpolymères utilisés selon l'invention sont généralement en dispersion aqueuse.

On peut utiliser notamment comme terpolymère, le terpolymère obtenu à partir d'acide acrylique, d'acrylate de méthyle et du composé de formule (III) où p est 40 et R2 est le radical béhényle. C'est le terpolymère acide méthacrylique/acrylate de méthyle/diméthyl m-isopropénylbenzylisocyanate d'alcool béhénylique éthoxylé à 40 OE, c'est-à-dire comportant 40 groupes oxyéthylénés.

Le terpolymère acrylique est présent dans les compositions de l'invention en une concentration en matière active d'au plus 0,5 % en poids par rapport au poids total de la composition. La concentration en matière active de terpolymère va de préférence de 0,01 à 0,5 % en poids et mieux de 0,15 à 0,3 % en poids par rapport au poids total de la composition.

Dans la composition selon l'invention, le rapport terpolymère/phase huileuse va de préférence de 1/30 à 1/100 et mieux de 1/50 à 1/100.

Selon un mode particulier de réalisation de l'invention, la composition de l'invention ne contient pas d'autres polymères que le terpolymère.

La phase huileuse de la composition selon l'invention représente généralement de 1 à 30 % et de préférence de 10 à 25 % en poids par rapport au poids total de la composition.

La nature de la phase huileuse de l'émulsion selon l'invention n'est pas critique. La phase huileuse peut ainsi être constituée par tous les corps gras et notamment les huiles, classiquement utilisés dans les domaines cosmétique ou dermatologique.

Parmi les huiles utilisables dans l'émulsion de l'invention, on peut citer par exemple les huiles végétales telles que les huiles de jojoba, avocat, amande douce, abricot, maïs et la fraction liquide de beurre de karité ; les huiles minérales comme l'huile de vaseline ; les huiles de synthèse comme le palmitate d'éthyl-2 hexyle, le myristate d'isopropyle, l'isoparaffine hydrogénée, l'isononanoate d'isononyle, l'octanoate de cétéaryle ; les huiles de silicone volatiles ou non volatiles ; et les huiles fluorées. Les autres corps gras susceptibles d'être présents dans la phase huileuse peuvent être par exemple les acides gras, les alcools gras, les cires et les huiles essentielles.

Selon un mode particulier de réalisation de l'invention, la composition de l'invention contient au moins une huile de silicone, de préférence une huile de silicone volatile qui peut être choisie par exemple parmi les polydiméthylsiloxanes cycliques ou linéaires, et leurs mélanges. Les polydiméthylsiloxanes cycliques ou cyclométhicones comportent d'environ 3 à 9 atomes de silicium, et de préférence de 4 à 6 atomes de silicium et peuvent être par exemple le cyclohexadiméthylsiloxane et le cyclopentadiméthylsiloxane. Les polydiméthylsiloxanes linéaires volatiles comportent de préférence d'environ 3 à 9 atomes de silicium. Les polydiméthylsiloxanes linéaires volatiles ont généralement une viscosité à 25°C inférieure ou égale à 5 cSt tandis que les cyclométhicones ont généralement une viscosité à 25°C inférieure ou égale à 10 cSt.

La composition selon l'invention contient un milieu physiologiquement acceptable, c'est-à-dire compatible avec la peau, les lèvres, le cuir chevelu, les yeux et/ou les cheveux.

De façon connue, les compositions de l'invention peuvent contenir des adjuvants habituels dans les domaines considérés, tels que des actifs hydrophiles ou lipophiles, des conservateurs, des antioxydants, des parfums, des solvants, des charges, des filtres, des matières colorantes, des agents basiques (triéthanolamine, soude) ou acides et encore des vésicules lipidiques. Ces adjuvants sont utilisés dans les proportions habituelles dans le domaine cosmétique, et par exemple de 0,01 à 30 % du poids total de l'émulsion, et ils sont, selon leur nature, introduits dans la phase aqueuse ou dans la phase huileuse de l'émulsion, ou encore dans des vésicules. Ces adjuvants ainsi que leurs concentrations doivent être tels qu'ils ne modifient pas la propriété recherchée pour l'émulsion.

Selon un mode particulier de l'invention, la composition de l'invention peut être exempte de nanopigments d'oxyde métallique.

Comme solvants, on peut citer par exemple les mono-alcools linéaires ou ramifiés ayant de 1 à 8 atomes de carbone comme l'éthanol, le propanol, le butanol, l'isopropanol, l'isobutanol ; les polyéthylène glycols ayant de 6 à 80 oxydes d'éthylène ; les polyols comme le propylène glycol, l'isoprène glycol et le butylène glycol.

Comme actifs, on peut citer par exemple les hydratants tels que les polyols comme la glycérine et le sorbitol ; les agents kératolytiques ; les dépigmentants ; les amincissants, et tout actif approprié pour le but final de la composition.

La composition de l'invention présente de préférence un pH qui respecte la peau et qui est compatible avec les terpolymères utilisés. Le pH de la composition va généralement de 6,5 à 8, de préférence de 7 à 7,5.

Les compositions selon l'invention peuvent se présenter par exemple sous forme de sérum ou de lait et sont préparées selon les méthodes usuelles. Elles ont l'avantage d'être vaporisables., ce qui signifie qu'elles sont assez fluides pour être appliquées au moyen d'un vaporisateur, lequel est exempt de gaz propulseur, est muni d'une pompe type pompe spray, fonctionne à la pression atmosphérique et délivre la composition sous forme de fines gouttelettes.

Les compositions, objets de l'invention, trouvent leur application notamment dans un grand nombre de traitements cosmétiques de la peau, des lèvres et des cheveux, y compris le cuir chevelu, notamment pour le soin, le démaquillage, le nettoyage et/ou le parfumage de la peau, des lèvres et/ou des cheveux.

Les compositions selon l'invention peuvent plus particulièrement être utilisées comme produits de soin et/ou de nettoyage pour le visage, comme produits parfumés pour la peau.

Aussi, l'invention a encore pour objet l'utilisation cosmétique de la composition telle que définie ci-dessus pour le soin, le démaquillage, le nettoyage et/ou le parfumage de la peau, des lèvres et/ou des cheveux.

L'invention a enfin pour objet un procédé de traitement cosmétique de la peau, y compris du cuir chevelu, des cheveux, et/ou des lèvres, caractérisé par le fait qu'on applique sur la peau, les cheveux et/ou les lèvres, une composition telle que définie ci-dessus.

Les compositions selon l'invention peuvent servir aussi pour l'imprégnation de tissus (tissés et non tissés) constituant des feuilles de nettoyage ou lingettes destinées au nettoyage et/ou au démaquillage de la peau, des cils et/ou des lèvres. Le tissu tissé ou non tissé peut être constitué de fibres naturelles ou synthétiques et par exemple de coton, polyamide, polyéthylène, polyester, polymère acrylique, rayonne, soie ou papier.

L'invention a donc aussi pour objet une feuille de nettoyage obtenue par imprégnation d'un tissu avec une composition telle que définie ci-dessus.

L'invention a aussi pour objet l'utilisation de la composition telle que définie ci-dessus pour la préparation d'une feuille de nettoyage destinée au démaquillage et/ou au nettoyage de la peau, des lèvres et/ou des cils.

Les exemples qui suivent permettront de mieux comprendre l'invention, sans toutefois présenter un caractère limitatif. Les quantités indiquées sont en % en poids, sauf mention contraire.

### Exemple 1 : Emulsion ultra-fluide vaporisable

| *Phase A* | |
|---|---|
| Terpolymère acide méthacrylique/acrylate de méthyle/diméthyl m-isopropénylbenzylisocyanate d'alcool béhénylique éthoxylé à 40 OE, en dispersion aqueuse à 24 % (soit 0,3 % de matière active) | 1,25 % |
| Soude | 0,06 % |
| Conservateurs | 0,2 % |
| Glycérine | 4 % |
| Eau déminéralisée | qsp 100 % |

| *Phase B* | |
|---|---|
| Huile d'abricot | 15 % |
| Cyclométhicone (cyclopentasiloxane) | 7 % |
| Parfum | 0,15 % |

Mode opératoire : On ajoute la soude au polymère sous agitation et on réalise l'émulsion en versant la phase B dans la phase A sous agitation. On effectue ensuite une homogénéisation sous pression (500 bars).

On obtient une émulsion très fluide, particulièrement adaptée pour le soin du visage pour les peaux grasses. La viscosité de l'émulsion est de 0,08 Pa.s (0,8 poises) au Rhéomat 180 muni d'une corps de mesure de type ancre, mobile 2. On peut également utiliser l'émulsion comme produit hydratant pour le corps et comme produit amincissant en vaporisation locale.

### Exemple 2 : Voile de parfum ultra fluide en spray

| *Phase A* | |
|---|---|
| Terpolymère acide méthacrylique/acrylate de méthyle/diméthyl m-isopropénylbenzylisocyanate d'alcool béhénylique éthoxylé à 40 OE, en dispersion aqueuse à 24 % (soit 0,3 % de matière active) | 1,25 % |
| Soude | 0,06 % |
| Conservateurs | 0,2 % |
| Glycérine | 4 % |
| Eau déminéralisée | qsp 100 % |

| *Phase B* | |
|---|---|
| Huile de jojoba | 7 % |
| Cyclométhicone (cyclopentasiloxane) | 12 % |
| Parfum | 3 % |

Le mode opératoire est identique à celui de l'exemple 1.

On obtient une émulsion très fluide ayant une viscosité de 0,1 Pa.s (1 poise), particulièrement adaptée pour le parfumage du corps. Sa pénétration est instantanée et ne laisse pas de film gras sur la peau.

## Revendications

1. Composition fluide sous forme d'émulsion huile-dans-eau comprenant dans un milieu physiologiquement acceptable, une phase huileuse dispersée dans une phase aqueuse, caractérisée par le fait qu'elle contient jusqu'à 0,5 % en poids de matière active par rapport au poids total de la composition, d'au moins un terpolymère acrylique obtenu à partir (a) d'un acide carboxylique à insaturation α,β-éthylénique, (b) d'un monomère à insaturation éthylénique non tensioactif différent de (a), et (c) d'un monomère uréthanne non-ionique qui est le produit de réaction d'un composé amphiphile non-ionique monohydrique avec un isocyanate à insaturation monoéthylénique.

2. Composition selon la revendication 1, caractérisée en ce qu'elle est exempte de tensioactif.

3. Composition selon la revendication 1 ou 2, caractérisée par le fait que le terpolymère acrylique comprend, par rapport au poids total du terpolymère, :
(a) environ de 20 à 70 % en poids, et de préférence de 25 à 55 % en poids, d'un acide carboxylique à insaturation α,β-éthylénique,
(b) environ de 20 à 80 % en poids, et de préférence de 30 à 65 % en poids, d'un monomère à insaturation éthylénique non tensioactif diifférent de (a), et
(c) environ de 0,5 à 60 % en poids, et de préférence de 10 à 50 % en poids, d'un monomère uréthanne non ionique qui est le produit de réaction d'un composé amphiphile non ionique monohydrique avec un isocyanate à insaturation monoéthylénique.

4. Composition selon l'une quelconque des revendications précédentes, caractérisée par le fait que l'acide carboxylique à insaturation α,β-monoéthylénique (a) est choisi parmi l'acide acrylique, l'acide méthacrylique, l'acide crotonique, l'acide itaconique et l'acide maléique.

5. Composition selon l'une quelconque des revendications précédentes, caractérisée par le fait que l'acide carboxylique à insaturation α,β-monoéthylénique (a) est l'acide méthacrylique.

6. Composition selon l'une quelconque des revendications précédentes, caractérisée en ce que le monomère à insaturation monoéthylénique non tensioactif (b) est choisi parmi les acrylates et méthacrylates d'alkyle en C₁-C₄, le styrène, le vinyltoluène, l'acétate de vinyle, l'acrylonitrile et le chlorure de vinylidène.

7. Composition selon l'une quelconque des revendications précédentes, caractérisée en ce que le monomère à insaturation monoéthylénique non tensioactif (b) est l'acrylate de méthyle ou l'acrylate d'éthyle.

8. Composition selon l'une quelconque des revendications précédentes, caractérisée en ce que le composé amphiphile non-ionique monohydrique utilisé pour obtenir le monomère uréthane non-ionique est un composé ayant la formule (I) suivante :
R-(OCH₂CHR')m- (OCH₂CH₂)n-OH (I)
dans laquelle R est choisi parmi les groupes alkyle comportant de 6 à 30 atomes de carbone et les groupes aralkyle comportant de 8 à 30 atomes de carbone, R' est choisi parmi les groupes alkyle comportant de 1 à 4 atomes de carbone, n est un nombre moyen allant d'environ 6 à 150 et m est un nombre moyen allant d'environ 0 à 50, à la condition que n soit au moins aussi grand que m et que n + m = 6 à 150.

9. Composition selon la revendication précédente, caractérisée en ce que dans le composé de formule (I), R est choisi parmi les groupes alkyle comportant de 18 à 26 atomes de carbone et alkyl-(C₈-C₁₃)-phényle ; le groupe R' est le groupe méthyle ; m = 0 et n = 6 à 150.

10. Composition selon l'une quelconque des revendications précédentes, caractérisée par le fait que le monoisocyanate à insaturation éthylénique utilisé pour former le monomère uréthanne non ionique (c) est l'α,α-diméthyl-m-isopropényl-benzylisocyanate.

11. Composition selon l'une quelconque des revendications précédentes, caractérisée par le fait que le terpolymère acrylique est obtenu par copolymérisation en dispersion aqueuse à partir de l'acide méthacrylique comme composant (a), de l'acrylate de méthyle comme composant (b) et d'un macromonomère uréthane non-ionique comme composant (c), ayant la structure (III) suivante : 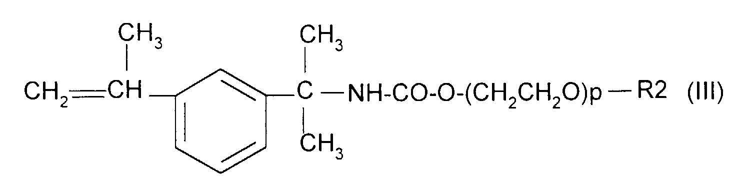 dans laquelle p va de 6 à 150 et R2 est choisi parmi les radicaux alkyle linéaires comportant de 18 à 26 et de préférence de 20 à 24 atomes de carbone.

12. Composition selon l'une quelconque des revendications précédentes, caractérisée par le fait que le terpolymère est présent en une concentration en matière active allant de 0,01 à 0,5 % en poids par rapport au poids total de la composition.

13. Composition selon l'une quelconque des revendications précédentes, caractérisée par le fait que la phase huileuse représente de 1 % à 30 % et de préférence de 10 à 25 % en poids par rapport au poids total de la composition.

14. Composition selon l'une quelconque des revendications précédentes, caractérisée en ce qu'elle contient au moins une huile de silicone volatile.

15. Composition selon l'une quelconque des revendications précédentes, caractérisée en ce qu'elle présente un pH allant de 6,5 à 8.

16. Utilisation cosmétique de la composition selon l'une quelconque des revendications 1 à 15, pour le soin, le démaquillage, le nettoyage et/ou le parfumage de la peau, des lèvres et/ou des cheveux.

17. Feuille de nettoyage obtenue par imprégnation d'un tissu avec une composition selon l'une quelconque des revendications 1 à 15.

18. Utilisation de la composition selon l'une quelconque des revendications 1 à 15 pour la préparation d'une feuille de nettoyage destinée au démaquillage et/ou au nettoyage de la peau, des lèvres et/ou des cils.

19. Procédé de traitement cosmétique de la peau, des cheveux, et/ou des lèvres, caractérisé par le fait qu'on applique sur la peau, les cheveux et/ou les lèvres, une composition selon l'une quelconque des revendications 1 à 15.
